# FASCICULE DE BREVET EUROPEEN

(11) **EP 1 023 230 B1**
(45) Date de publication et mention de la délivrance du brevet: **24.07.2002**
(21) Numéro de dépôt: 98949043.8
(22) Date de dépôt: 13.10.1998
(51) Int. Cl.: B65D 83/14, F16L 23/22, A61K 9/00

(54) **JOINT D'ETANCHEITE POUR UNE VALVE**
DICHTUNG FÜR EIN VENTIL
VALVE SEALING JOINT

(30) Priorité: 13.10.1997 FR 9712773; 16.01.1998 FR 9800440
(43) Date de publication de la demande: 02.08.2000
(73) Titulaire: VALOIS S.A., 27110 Le Neubourg (FR)
(72) Inventeur: TCHEREVATCHENKOFF, André, F-63500 Issoire (FR)
(74) Mandataire: CAPRI SARL
(86) Numéro de dépôt international: FR9802186
(87) Numéro de publication internationale: WO9919235

(56) Documents cités:
- WO-A-95/17195
- DE-A- 3 927 787
- GB-A- 2 246 605

## Description

La présente invention concerne un joint de valve et plus particulièrement un joint de col destiné à réaliser l'interface étanche entre ladite valve et le col du récipient sur lequel la valve est montée. On peut également utiliser un joint conforme à l'invention en tant que joint de soupape dans une valve. A vrai dire, l'invention s'applique à tout joint destiné à venir en contact avec un produit fluide tel qu'un propulseur apte à extraire des composés inclus dans ledit joint. L'invention trouvera donc une application privilégiée dans le domaine du conditionnement de certains produits pharmaceutiques sous forme de pulvérisateurs, dans lesquels le produit pharmaceutique ne doit pas être pollué par des composants polluants extraits des joints de la valve par le propulseur.

En effet, un joint de valve, et en particulier le joint de col de la valve, doit satisfaire à plusieurs exigences. Premièrement, il doit assurer une bonne étanchéité au propulseur contenu dans le récipient avec le produit fluide à distribuer. Ensuite, il doit assurer une bonne étanchéité à la vapeur d'eau provenant de l'atmosphère et qui pénètre dans le récipient. Afin d'éviter l'agglomération et/ou l'hydrolyse des particules actives, le taux de vapeur d'eau dans le propulseur devrait être minimal, et notamment maintenu à une valeur inférieure à 300 ppm pour un propulseur tel que du HFA (hydro-fluoro-alcane) avec ou sans éthanol. Enfin, le joint de pompe ne doit pas relarguer trop de composés qu'il contient et qui sont connus sous le terme d' "extractibles". Ces extractibles sont des composés extraits du joint par le propulseur qui contaminent ou polluent le produit fluide à distribuer. Parmi ces extractibles, on distingue deux catégories : tout d'abord, des toxiques tels que des nitrosamines, des polynucléaires aromatiques, mais aussi des monomères. Ces toxiques sont en général en faibles quantités : nitrosamine (ppb), polynucléaires aromatiques (ppm), monomères (ppb). Ils sont donc rares. Mais il y a surtout des produits dits majoritaires que l'on retrouve en plus forte concentration. Parmi ces produits majoritaires, on trouve des antioxydants, des émulsifiants, des oligomères et parfois des plastifiants. Les oligomères sont constitués de bas poids moléculaires et se trouvent surtout dans les thermoplastiques élastomères TPE.

Le document GB-2 246 605 divulgue un joint tri-couche comportant une couche centrale rigide pour éviter la déformation du joint lors du montage et deux couches externes souples pour réaliser l'étanchéité, ces couches externes pouvant être réalisées en un composé polyéthylène-butyle.

Le but de la présente invention est de pallier les inconvénients de l'art antérieur en définissant un joint facile et économique à fabriquer, à découper et à distribuer, présentant d'excellentes qualités d'étanchéité et qui ne relargue que très peu de polluants tels que des oligomères, voire des plastifiants.

La présente invention a pour objet un joint de valve destiné à venir en contact avec un produit, tel qu'un propulseur ou une solution du type propulseur/solvant, le joint étant réalisé à partir d'un mélange butyle-polyéthylène, le rapport dudit mélange étant situé dans le domaine compris entre environ 2:3 et environ 3:2, c'est-à-dire que le mélange peut varier entre environ 40% de butyle et 60% de polyéthylène, et environ 60% de butyle et 40% de polyéthylène.

Le polyéthylène forme une bonne barrière contre l'extraction par un propulseur tel que du HFA et de plus présente un faible coefficient de frottement ce qui rend les joints plus faciles à découper et à distribuer.

D'autre part, un joint réalisé à partir d'un mélange butyle-polyéthylène confère d'excellentes qualités d'étanchéité à la fois à la vapeur d'eau et aux propulseurs. Avec un rapport situé dans la plage ci-dessus, toutes les exigences ci-dessus sont remplies de manière optimale.

Selon une première variante avantageuse, le mélange butyle-polyéthylène est réalisé dans un rapport 42,5:57,5.

Selon une deuxième variante avantageuse, le mélange butyle-polyéthylène est réalisé dans un rapport 1:1.

Selon une troisième variante avantageuse, le mélange butyle-polyéthylène est réalisé dans un rapport 57,5:42,5.

Avantageusement, le mélange butyle-polyéthylène est basse densité.

En particulier, le joint peut être utilisé en tant que joint de col formant interface étanche entre ladite valve ou pompe et le col du récipient sur lequel ladite valve ou pompe est montée.

Avantageusement, le joint peut être utilisé dans un dispositif de distribution aérosol d'un médicament pour le traitement de l'asthme.

L'invention sera maintenant décrite plus amplement en décrivant un procédé de fabrication de joints selon l'invention.

On commence par la fabrication de bandes en utilisant un matériau souple apte à former le joint. En tant que matériau de l'invention, on utilise un mélange butyle/polyéthylène, de préférence basse densité qui fait partie de la famille des thermoplastiques élastomères TPE. Dans l'exemple suivant, il sera fait référence au TPE à titre de simplification. Les bandes de TPE sont obtenues par extrusion à travers une extrudeuse qui pousse le TPE en fusion à travers une filière en forme de fente allongée. A la sortie, la bande non finie est laminée entre des rouleaux de laminage pour lui conférer l'épaisseur voulue.

Le polyéthylène présente de bonnes caractéristiques d'effet barrière à l'extraction des composés polluants contenus dans le joint. De plus, il possède un faible coefficient de frottement, ce qui facilite la découpe de la bande et la distribution des joints ainsi découpés, si la concentration de polyéthylène n'est pas inférieure à environ 40%.

De tels joints peuvent être utilisés en tant que joint de col formant l'interface étanche entre la valve et le col du récipient sur lequel elle est montée. On peut également utiliser des joints ainsi fabriqués en tant que joint de soupape dans des valves.

Les performances des joints de l'invention ont été testées, à titre d'exemple, dans une application de joint de col pour valve doseuse fonctionnant avec un gaz HFA-134a en tant que propulseur. L'épaisseur de ces joints a été établie entre 1 et 1,5 mm, et plusieurs mesures ont été réalisées. Ainsi, le taux de fuite a été mesuré à température ambiante et à 40°C, puis extrapolé à une valeur donnée en mg/an. D'autre part, l'entrée d'humidité, c'est-à-dire le taux de vapeur d'eau dans le propulseur provenant de l'atmosphère, donné en ppm, a été mesurée deux fois, à un mois d'intervalle. Les résultats sont listés dans la tableau ci-dessous, avec à titre d'exemples comparatifs, les performances en conditions similaires d'autres matériaux de joints connus, comprenant le nitrile, le chloroprène, un mélange EPDM-polypropylène et un mélange butyle-polypropylène.

**TABLEAU**

| matériau de joint | taux de fuite (mg/an) | | humidité (ppm) | |
|---|---|---|---|---|
| | temp.amb | 40°C | t=0 | t=1mois |
| butyle-polyéthylène 42,5:57,5 | 12 | 38 | 230 | 239 |
| | | | | |
| butyle-polyéthylène 50:50 | 14 | 37 | 172 | 270 |
| | | | | |
| butyle-polyéthylène 57,5:42,5 | 11 | 34 | 180 | 261 |
| | | | | |
| nitrile | 96 | 468 | 250 | 350 |
| | | | | |
| chloroprène | 60 | 95 | 168 | 230 |
| | | | | |
| EPDM-polypropylène 50:50 | 300 | 1 800 | 200 | 240 |
| | | | | |
| butyle-polypropylène 50:50 | 26 | 107 | 180 | 210 |

D'après les résultats ci-dessus, il apparait que les mélanges butyle-polyéthylène de l'invention sont supérieurs au nitrile pour le taux de fuite et l'entrée d'humidité, et supérieurs au chloroprène et aux mélanges EPDM-polypropylène et butyle-polypropylène pour le taux de fuite.

Le matériau de l'invention fournit donc des performances optimales, tout en étant facile et économique à fabriquer.

Les résultats sont relativement constants sur l'ensemble du domaine de concentration du mélange butyle-polyéthylène de l'invention. Le mélange à un rapport de 57,5% de butyle et 42,5% de polyéthylène présente les meilleurs résultats concernant le taux de fuite, alors que le mélange à un rapport de 42,5% de butyle et 57,5% de polyéthylène présente les meilleurs résultats concernant l'entrée d'humidité. Bien que non représenté sur le tableau, les performances diminuent lorsque ces concentrations sortent du domaine de l'invention.

Ces performances inférieures combinées avec une difficulté et donc un coût de fabrication supérieur, lorsque le rapport du mélange butyle-polyéthylène est situé hors du domaine compris entre environ 2:3 et 3:2, montrent que le domaine revendiqué procure des joints optimaux, tant au niveau des performances d'étanchéité que du coût de fabrication.

## Revendications

1. Joint de valve destiné à venir en contact avec un produit, tel qu'un propulseur ou une solution du type propulseur/solvant, le joint étant réalisé à partir d'un mélange butyle-polyéthylène, **caractérisé en ce que** le rapport dudit mélange est situé dans le domaine compris entre environ 2:3 et environ 3:2.

2. Joint selon la revendication 1, dans lequel le mélange butyle-polyéthylène est réalisé dans un rapport 42,5:57,5.

3. Joint selon la revendication 1, dans lequel le mélange butyle-polyéthylène est réalisé dans un rapport 1:1.

4. Joint selon la revendication 1, dans lequel le mélange butyle-polyéthylène est réalisé dans un rapport 57,5:42,5.

5. Joint selon l'une quelconque des revendications précédentes, dans lequel le mélange butyle-polyéthylène est de préférence basse densité.

6. Joint selon l'une quelconque des revendications précédentes, utilisé en tant que joint de col formant interface étanche entre ladite valve ou pompe et le col du récipient sur lequel ladite valve ou pompe est montée.

7. Joint selon la revendication 6, utilisé en tant que joint de col dans une valve doseuse fonctionnant avec un gaz propulseur du type HFA-134a, HFA-227 ou avec un gaz HFA mélangé avec un solvant, tel que de l'éthanol.

8. Joint selon l'une quelconque des revendications précédentes, utilisé dans un dispositif de distribution aérosol d'un médicament pour le traitement de l'asthme.

## Claims

1. A valve seal intended to come into contact with a fluid such as a propellant or a propellant/solvent type solution, the seal being produced from a butyl rubber and polyethylene mixture, the seal being **characterized in that** the proportions of said mixture are in a ratio in the range between about 2:3 and about 3:2.

2. A seal according to claim 1, in which the ratio of the butyl rubber to the polyethylene in the mixture is 42.5:57.5.

3. A seal according to claim 1, in which the ratio of the butyl rubber to the polyethylene in the mixture is 1:1.

4. A seal according to claim 1, in which the ratio of the butyl rubber to the polyethylene in the mixture is 57.5:42.5.

5. A seal according to any preceding claim, in which the butyl rubber and polyethylene mixture is preferably of low density.

6. A seal according to any preceding claim, used as a neck seal forming an impervious interface between said valve or a pump and the neck of the receptacle on which said valve or pump is mounted.

7. A seal according to claim 6, used as a neck seal in a metering valve operated with an HFA-134a, HFA-227 type propellant gas or with an HFA type gas mixed with a solvent such as ethanol.

8. A seal according to any one of the preceding claims, used as a device for distributing an aerosol of medication for treating asthma.

## Patentansprüche

1. Ventildichtung, die dazu dient, mit einem Produkt, wie zum Beispiel einem Treibmittel oder einer Lösung die aus einem Treibmittel und einem. Lösemittel besteht in Berührung zu kommen, wobei die Dichtung ausgehend von einer Butyl-Polyethylen-Mischung hergestellt wird, **dadurch gekennzeichnet, daß** sich das Verhältnis dieser Mischung in einem Bereich zwischen ungefähr 2:3 und ungefähr 3:2 befindet.

2. Dichtung nach Anspruch 1, bei der die Butyl-Polyethylen-Mischung ein Verhältnis von 42,5:57,5 besitzt.

3. Dichtung nach Anspruch 1, bei der die Butyl-Polyethylen-Mischung ein Verhältnis 1:1 besitzt.

4. Dichtung nach Anspruch 1, bei der die Butyl-Polyethylen-Mischung ein Verhältnis von 57,5:42,5 besitzt.

5. Dichtung nach einem der vorhergehenden Ansprüche, bei der die Butyl-Polyethylen-Mischung vorzugsweise eine niedrige Dichte besitzt.

6. Dichtung nach einem der vorhergehenden Ansprüche, die als Hals-Dichtung Verwendung findet, die ein dichtes Zwischenelement zwischen dem Ventil oder der Pumpe und dem Hals des Behälters bildet, auf dem das Ventil oder die Pumpe montiert ist.

7. Dichtung nach Anspruch 6, die Verwendung als Hals-Dichtung in einem Dosierventil Verwendung findet, das mit einem Treibgas des Typs HFA-134a, HFA-227 oder mit einer Mischung eines HFA-Gases mit einem Lösemittel wie zum Beispiel Ethanol arbeitet.

8. Dichtung nach einem der vorhergehenden Ansprüche, die in einer Aerosol-Abgabevorrichtung für ein Medikament zur Behandlung von Asthma Verwendung findet.
